# EUROPEAN PATENT APPLICATION

(11) **EP 4 035 695 A1**
(43) Date of publication of application: **03.08.2022**
(21) Application number: 21154261.8
(22) Date of filing: 29.01.2021
(51) Int. Cl.: A61L 9/015, A61L 9/12, A61M 15/00, B05C 5/02

(54) **AIR INFUSION APPARATUS**

(71) Applicant: Philipp, Harald, 8008 Zürich (CH)
(72) Inventor: Philipp, Harald, 8008 Zürich (CH)
(74) Representative: Keilitz Haines & Partner Patentanwälte PartGmbB

(57) **Abstract**

An apparatus (1, 50) for expressing a trace gas, such as a fragrance or disinfectant, from an elongate carrier medium (4). The carrier medium (4) is passed through an activation zone where the medium is energized by a heat source or an electromagnetic radiation source to liberate trace gas from the medium. The trace gas so expressed is further conveyed via a transport gas in an air flow (15, 54, 59) to a desired destination such as to a volume of air in a room. The apparatus may include a sensor (62) for sensing the level of gas, so as to permit regulation of the concentration of the trace gas to a suitable level.

## Description

### FIELD OF THE INVENTION

The present disclosure relates to apparatus for introducing a trace gas into an ambient environment from a consumable carrier medium.

### BACKGROUND

It can be desirable to infuse a trace gas into the air for various purposes. One such purpose is to mask odors, i.e. an air freshener. Another purpose is to create a sense of well-being among persons inhabiting the space. The use of fragrances and masking agents is well known for centuries, for example incense, floral extracts and various fruits and herbs. Another purpose is to introduce a disinfecting agent, for example a halogen gas or compound which causes aerosolized biological agents to become rapidly denatured. An example is to infuse trace concentrations of vaporized atomic halogen gas as described in a paper by Dr F. M. Burnet and Dr J. D. Stone "The action of halogens on influenza virus with special reference to the action of iodine vapour on virus mists", PMID: 21006104 DOI: 10.1038/icb.1945.32, where the trace concentrations are at sub parts per million (PPM) levels.

The use of a solid form of carrier for the substance to be infused into the air is desirable to provide for convenient consumer operation. Whereas a liquid form of substance is subject to spillage, staining, transportation problems and other inconveniences, a solid form can provide for a safe and concentrated form of delivery of a trace gas.

### BRIEF SUMMARY OF THE INVENTION

According to one aspect of the invention there is provided a trace gas emission apparatus for processing a substance-bearing, strip-format carrier to activate the substance and thereby release a trace gas, the apparatus comprising:
a substance activator defining an activation zone dimensioned to accommodate a length portion of a strip-format carrier, the substance activator being configured to energize a length portion of a carrier situated in the activation zone and thereby activate substance in that length portion; and
a feed mechanism configured to feed a strip-format carrier through the activation zone from one end of the strip-form carrier to another, thereby to enable the substance activator to effect incremental release of trace gas from respective length portions of the strip-format carrier.

In some embodiments, the substance activator comprises an electromagnetic radiation emitter operable to direct an electromagnetic radiation beam to the activation zone.

In other embodiments, the substance activator comprises a heater arranged to apply heat in the activation zone. The heater may comprise a hot gas source operable to direct a hot carrier gas stream to the activation zone. The substance activator may comprise a roller arranged in the activation zone to come into contact with a length portion of the strip-format carrier, wherein the heater is operable to heat the roller so that substance activation takes place by heat transfer from the roller to a contacted length portion of the strip-format carrier. A further roller may be provided that is arranged with said roller to form a counter-rotating roller pair defining a nip region, wherein the feed mechanism is configured to convey the strip-format carrier through the nip region. The heater may be operable to heat the further roller. The feed mechanism may comprise a rotary drive connected to drive at least one of the roller and the further roller, so that the counter-rotating roller pair fulfills a dual function for both the feed mechanism and the substance activator.

In certain embodiments suitable for both electromagnetic radiation source or heat activated embodiments, the feed mechanism comprises a roller and a further roller arranged to form a counter-rotating roller pair defining a nip region and a rotary drive connected to drive at least one of the roller and the further roller so as to convey the strip-format carrier through the nip region. The substance activator may comprise at least one spike or blade operable to pierce or cut into a surface of the strip-format carrier to assist release of substance.

In certain embodiments, the feed mechanism is configured to feed a flexible tape as the strip-format carrier, and the substance activator is configured to activate the substance from a length portion of the flexible tape.

In certain embodiments, the feed mechanism is configured to feed a self-supporting stick as the strip-format carrier, and the substance activator is configured to activate the substance from a length portion of the self-supporting stick.

A controller for the apparatus may be provided. The controller may be configured to control at least one of the substance activator and the feed mechanism so that the trace gas is emitted at a controlled rate. To assist operation of the controller, a sensor may be provided. The sensor is configured and arranged to measure concentration of the trace gas and to supply a sensor signal indicative thereof to the controller, the controller being configured to control at least one of the substance activator and the feed mechanism responsive to the sensor signal so as to maintain the concentration of the trace gas at a desired level.

According to another aspect of the invention there is provided a tape dispensing cartridge comprising:
a tape wound in a coil, the tape storing at least one substance capable of producing a trace gas, wherein the at least one substance is retained bound to the tape in ambient conditions and in response to being locally activated emits the trace gas; and
a shell in which the coil of tape is rotatably arranged, the shell having an aperture from, or past, which the tape is conveyable under rotation of the coil.

The cartridge may further comprise a dispensing reel that is rotatably mounted in the shell and fixed to one end of the tape. The cartridge may further comprise a return reel that is rotatably mounted in the shell and fixed to another end of the tape, such that the tape can be wound from the dispensing reel onto the return reel, the tape having a length portion situated between the dispensing reel and the return reel that lies at the aperture. The tape may additionally bear a further substance, said substance and said further substance being arranged side-by-side along the tape to form first and second tracks that run parallel to one another.

According to another aspect of the invention there is provided a gas dispenser unit for dispensing a trace gas into an environment, the apparatus comprising:
an air inlet providing gas communicative access to the apparatus from its surroundings;
an air outlet providing gas communicative access from the apparatus to its surroundings;
a fan which generates an air flow through the apparatus along a flow path from the air inlet to the air outlet; and
a trace gas emission apparatus as described above arranged to emit trace gas into the flow path generated by the fan so that air emitted from the air outlet is infused with the trace gas.

The gas dispenser unit may further comprise a trace gas dispensing conduit having an inlet aperture arranged to receive trace gas emitted from the trace gas emission apparatus and an outlet aperture arranged to emit trace gas into the air flow ahead of the fan.

Several mechanisms of gas expression are contemplated for use in embodiments of the invention. These are:
- Single transition sublimation for the creation of a gas from an element or compound in a solid state;
- Single transition evaporation for the creation of a gas from a substance in a liquid state;
- Double transition evaporation, whereby a solid substance which is converted first into a liquid and then the liquid is evaporated;
- Chemical reaction between two substances which when mixed, for example under heat or when exposed to specific wavelengths of electromagnetic radiation, create a desired gas;
- Chemical dissociation whereby a compound when heated or irradiated dissociates into two or more of its constituent components, at least one of which is emitted as a gas; and
- Direct emission of a gas from a container, where the container comprises encapsulation, and more specifically micro-encapsulation of an element or compound in the gaseous, liquid or solid state, where the encapsulation is breached mechanically, by heating, or by irradiation.

For clarity and brevity in this disclosure, the words 'express' or 'expression' or 'emit' or 'emission' will be used to encompass all the foregoing examples of trace gas generation.

Certain embodiments of the invention may provide for the expression of a trace gas using as a medium a solid-state flexible ribbon or tape, or elongate semi-rigid or rigid stick. The medium is impregnated or coated with an expressible substance which when heated by a heater or irradiated with electromagnetic radiation such as from a laser, releases the desired gas or gases into a transport gas such as ambient air. Such a ribbon, tape or stick is inserted into an apparatus which acts to feed the ribbon, tape or stick into an activator. The activator in one embodiment comprises a heated element that comes into contact or proximity with a portion of the medium and creates a local hot zone at that portion of the medium. The activator in another embodiment is a hot gas source operable to generate a directed hot carrier gas stream onto a portion of the medium. The activator in another embodiment comprises an electromagnetic radiation source, such as a laser, arranged to direct an electromagnetic radiation beam, such as a laser beam, onto a portion of the medium.

In case of a ribbon-form medium, a cartridge dispenser may be employed comprising a cartridge casing in which the ribbon is contained as a coil. One suitable form of cartridge is a supply-only cartridge in which an end of the ribbon is fed out gradually to the substance activator. After passage through the substance activator, the consumed ribbon portion, i.e. that part that has been expressed, can be received by a separate waste container. A second suitable form of cartridge has a take-up partition or zone within the cartridge into which the ribbon is fed post-expression; upon sufficient consumption of the ribbon, the cartridge may be removed from the expression apparatus and disposed of or recycled.

In the case of a stick-form medium, where the stick carrying the medium is generally preferred to be semi-rigid or rigid, the stick can be inserted into the apparatus at an entry port and expelled once it is consumed, either as a whole stick or incrementally as the stick is incrementally expressed. The expressed stick or stick portions can be expelled to a suitably positioned waste canister.

The apparatus may infuse air with two or more trace gases. For example, release of a fragrance may be combined with release of a disinfecting agent.

### BRIEF DESCRIPTION OF THE DRAWINGS

In the following detailed description, for purposes of explanation and not limitation, specific details are set forth in order to provide a better understanding of the present disclosure. It will be apparent to one skilled in the art that the present disclosure may be practiced in other embodiments that depart from these specific details.
Figures 1a and 1b are schematic front and side views of a first example trace gas emission apparatus which consumes a flexible ribbon medium by means of at least one heated roller.
Figures 2a and 2b are schematic front and side views of a second example trace gas emission apparatus which consumes a flexible ribbon medium by means of an electromagnetic radiation source such as a laser diode.
Figure 3a and 3b are schematic front and side section views of a first example ribbon medium of indeterminate length, the ribbon medium comprising porous material infused with an expressible substance or substances and an expression zone thereon.
Figures 4a, 4b, and 4c are schematic views of a second example ribbon medium which is devised from an inner cavity layer containing the required expressible substances, with an outer protective envelope. Figures 4b and 4c are schematic front and side section views of the second example ribbon medium, and Figure 4a is a schematic side section view during fabrication of the ribbon medium.
Figure 5 is a schematic front view of a two-stripe ribbon medium containing two different expressible substances in different regions which can be expressed without direct mixing at the time of their simultaneous expression.
Figures 6a and 6b are schematic section and perspective views of a supply-only cartridge according to a first example cartridge, wherein this cartridge type does not take back consumed portions of a ribbon medium.
Figure 6c is a schematic front view of a ribbon or stick medium 4 showing how additional markings may be provided to encode information that may be used for process control.
Figures 7a and 7b are schematic front and side section views of a stick medium that is semi-rigid or rigid.
Figure 8 is a schematic side section view of a third example trace gas emission apparatus which is designed to accept a semi-rigid or rigid stick medium, the trace gas emission apparatus comprising an activator based on a pair of counter-rotating rollers, and the trace gas emission apparatus including a waste container arranged to accept spent stick media after they have passed through the activator.
Figure 9 is a schematic side section view of a pair of counter-rotating rollers as may be used in the trace gas emission apparatus of Figure 8, whereby the rollers contain projections for piercing the medium to allow for enhanced expression of the desired trace gas.
Figures 10a and 10b are schematic front and side views of a first example air infusion apparatus employing the trace gas emission apparatus of Figures 1a and 1b.
Figures 11a and 11b are schematic front and side views of a second example air infusion apparatus employing the trace gas emission apparatus of Figure 8.
Figure 12a is a schematic front section view of a second example cartridge based on a modification to an audio cassette with first and second reels. A ribbon-format medium is initially wound on a first reel, and in use is fed through a trace gas emission apparatus based on a heated roller pair, the expressed ribbon-format medium then being wound onto a second reel.
Figure 12b is a schematic front section view of a third example cartridge, which may be considered to be a modification of the design of Figure 12a, in which the trace gas emission apparatus is based on an electromagnetic radiation source instead of heated rollers.
Figures 13a and 13b are schematic front section and side section views of an example trace gas emission apparatus for the cartridge of Figure 12b but which may be adapted for the cartridge of Figure 12a.
Figures 14a and 14b are schematic front and side views of a third example trace gas emission apparatus incorporating the apparatus of Figures 13a and 13b, or alternately, a variation of that shown in 13a and 13b using at least one heated roller as *per* Figure 12a. Figure 14b also shows a remote sensor and a remote user controller, which may also be app based, as is common with today's mobile technology.

### DETAILED DESCRIPTION

Certain terms used in this disclosure are defined as follows:
**Express or Expression:** The act of causing the emission of a trace gas from a substrate (the medium) by any mechanism, particularly but not limited to stimulation via electromagnetic radiation or direct or indirect heating via a heated surface in contact with the medium, or a heated stream of gas e.g. air.
**Substance:** A single substance or combination of substances which are bound to a medium stably in ambient conditions and which, singly or in combination, cause release of a gas (the trace gas) from the medium when it is heated or irradiated with electromagnetic radiation. The chemical or physical mechanism by which the substance or substances cause release of the gas include: sublimation, evaporation, chemical reaction, dissociation (e.g. photodissociation) and chemical decomposition; additionally the mechanism can include a two-stage process of liquification (e.g. melting or softening) followed by evaporation.
**Medium:** A solid-state carrier material comprising a flexible or rigid substrate into or onto which the substance is incorporated or deposited during manufacture. The substance may be incorporated, e.g. impregnated, into the medium and/or deposited on a surface thereof. The medium may be porous for impregnation, or non-porous for surface deposition. A non-porous surface may be non-planar, e.g. exhibit surface roughness or structure, to increase the surface area and thereby enhance the amount of substance and/or the adhesion of the substance to the surface. The medium may also contain an inner cavity which contains the substance in bulk, the inner cavity for example being formed as the 'filling' in a sandwich between two jacketing layers.
**Trace Gas:** The gas released from the carrier when the medium is heated, irradiated or otherwise activated.
**Transport Gas:** The gas that flows past the medium as it is being expressed and which is used to convey the trace gas from the trace gas emission apparatus to an exit port of the apparatus for infusion into an ambient region or space. The transport gas may, for example, be air (ambient air or synthetic air) or an inert gas such as nitrogen, helium or argon.
**Expressor (Unit):** An electromechanical device which takes as an input a medium for the purpose of the expression of trace gas from said medium. The expressor unit is commonly a sub-assembly within a larger apparatus, the larger apparatus further incorporating a gas (air) handling assembly as well as standard components such as a user interface, a power supply, and so on.
**Ribbon Medium:** A type of medium which is flexible, having an arbitrary length and a fixed width, allowing it to be wound up in a coil, such as onto a hub or reel in the manner of a magnetic tape, where the reel may conveniently be contained within a cartridge or cassette. A ribbon medium allows a great substance capacity owing to the theoretically unbounded length. The width is dimensioned to match an expressor unit designed to accept such ribbon media.
**Stick Medium:** A type of medium which is generally inflexible, so as to be self-supporting, having a limited length and a fixed width. A stick medium has a limited substance capacity but is easy to handle and requires no container. The width is dimensioned to match an expressor unit designed to accept such stick media. Compared to a ribbon medium, a stick medium may be regarded as a short ribbon medium that is made inflexible, e.g. through greater thickness, so that it is self-supporting and can be handled directly. A stick medium may for example have the format of a tongue-depressor (spatula). A stick medium may be rigid or semi-rigid. In the latter case, the degree of rigidity should be sufficient that the stick medium is self-supporting, e.g. can be picked up by one end so as to allow the other end to be inserted into the input aperture of an expressor unit.

Disclosed herein are various versions of an apparatus suitable to the expression of a trace gas from all the above-stated media types and formats. The apparatus in combination with the medium can operate in a convenient and consumer-friendly way. In the following by way of example, the apparatus has the form of a floor-standing unit, which has the general format of a room air cleaner.

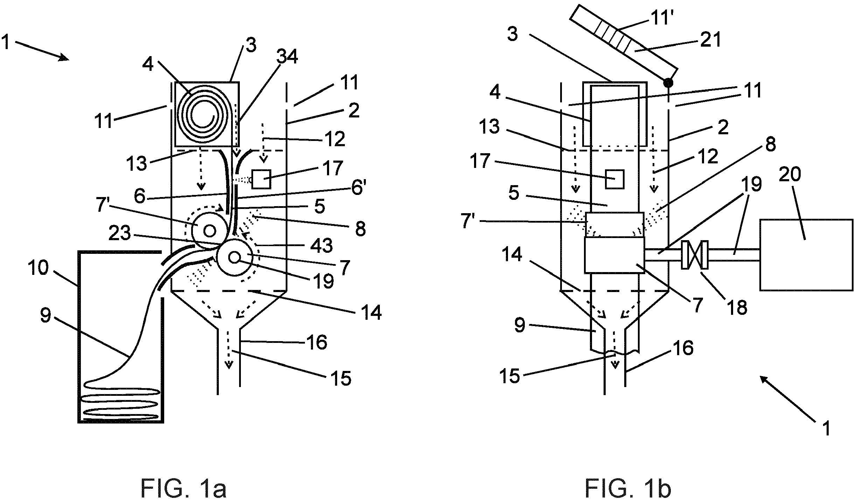

**Figures 1a and 1b** are schematic front and side views of a first example expressor unit 1. The expressor unit 1 is enclosed in housing 2, which is designed to accept a ribbon medium 4 contained in a cartridge shell 3. The medium travels in a direction 34, following a path 5 between guides 6 and 6' to a pair of counter-rotating rollers 7 and 7' with the direction of rotation being illustrated with arrow 43. The rollers 7, 7' form a nip region 23, which may have a gap dimensioned to be slightly less than the thickness of the ribbon medium, thereby to provide a feed mechanism. Alternatively, one or both of the rollers may be spring-loaded to bias one against the other, so that, when no ribbon medium is present, the rollers are in contact with each other, but when a ribbon medium is inserted, a gap in the nip region opens up and is maintained at the thickness of the ribbon medium by spring action. At least one of the rollers 7 or 7' is heated, preferably electrically. The use of electrically heated rollers is well known in the art. It is beneficial for the roller temperature of either or both rollers to be continually measured and controlled within a tolerance parameter to facilitate release of the trace gas from the ribbon medium at a desired rate. Roller temperature may be sensed, for example, via a thermocouple or thermistor or far-infrared detector (not shown).

The rate of rotation of the rollers 7 and 7' determine the linear consumption rate of the medium 4, but this is not completely determinative of the emission rate of trace gas 8. The emission rate of the trace gas 8 also depends on several other parameters including: the concentration of trace-gas generating substance on or in the medium, the net effective thickness of the substance on or in the medium, which determines the areal density of the substance, and the temperature of the rollers. The emission rate of the trace gas 8 is also affected by the flow of the transport gas 12, e.g. its density, temperature and flow speed, as it flows past the rollers 7, 7' and onward to an outlet conduit 16 as flow 15.

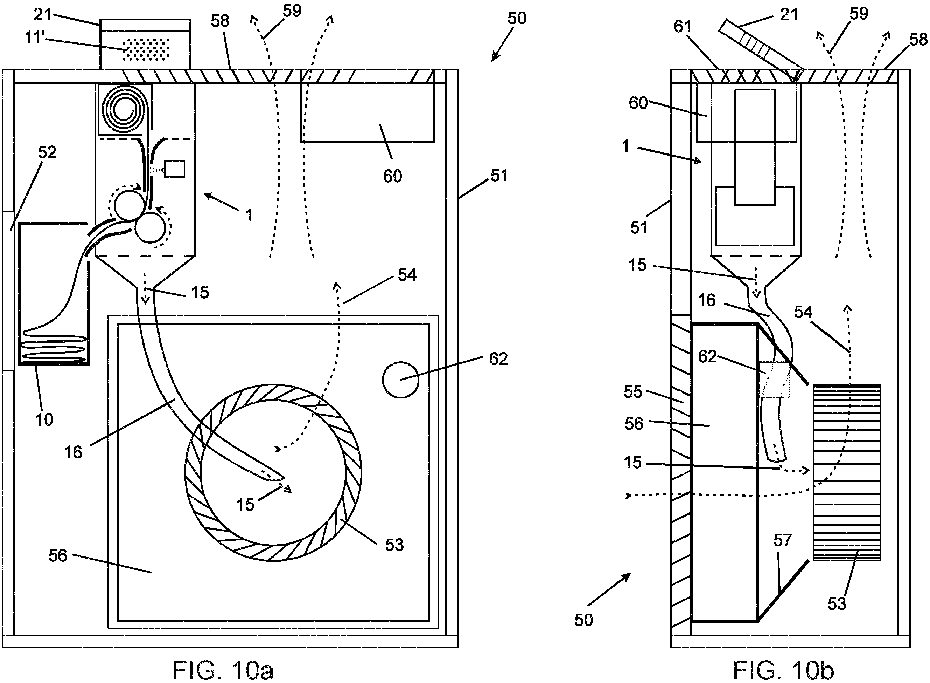

Figures 1a and 1b also show transport gas inlet port(s) 11 and 11', the latter contained within a lid 21, to allow for the transport gas (e.g. air) to flow to and past the expressor during operation. A horizontal support element 13 provides a resting place for cartridge 3, the support being perforated to allow for flow of the transport gas. A reflective photodetector 17 detects markings on the medium 4 as it runs through guides 6 and 6' so as to communicate with controller logic (see item 60 in Figure 10a) allowing controller logic to obtain and process information from the medium, such as medium type, substance type, start and stop markings on the medium, date and manufacture coding of the medium, and so on. At least one of the rollers 7 or 7' is driven by a motor 20 via a shaft 19, preferably through a heat-isolating coupling 18, for example a high temperature flexible polymer coupling, of which many are commercially available. The motor 20 preferably is of a digitally controlled type such as a stepper motor. Below the rollers there is a perforated grill or grating 14 which prevents matter from falling into the outlet conduit 16. Such matter may arise for example from feed errors, i.e. tearing of the ribbon. After exiting the rollers 7, 7', the depleted ribbon 9 is led to a waste container 10, which may from time to time be emptied by the user. If the container 10 is made of an inexpensive biodegradable material such as cardboard or a starch-based polymer, the entire container may be conveniently disposed of at little additional cost, thereby obviating the need for the user to come into contact with the depleted ribbon 9. Outlet conduit 16 provides a pathway for trace-gas laden transport gas 15 to the larger apparatus as will be described further below.

It should be clear from the foregoing that the transport gas flow, being shown in a downward direction via suction action from below, prevents the trace gas from backing up into the user-accessible cavity between plate 13 and the opening capped by lid 21. This airflow reduces the probability that the user could come into contact with concentrated trace gas, which at high levels could be noxious to the user. However, the unit 1 could be inverted or placed on its side with no change in performance. The use of suction to facilitate transport gas flow can also be replaced by, or supplemented with, forced transport gas entry from the cartridge end.

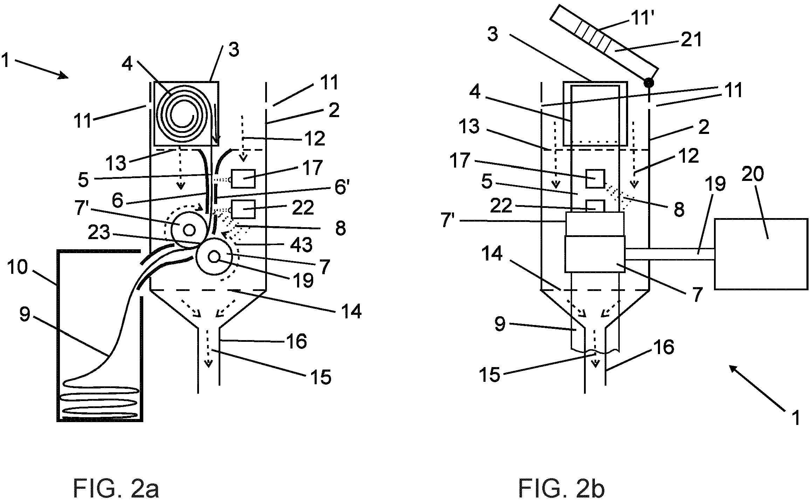

**Figures 2a and 2b** are schematic front and side views of a second example expressor unit 1 which consumes a flexible ribbon medium by means of an electromagnetic radiation source such as a laser diode. Comparing with Figures 1a and 1b, Figures 2a and 2b have the same basic configuration and use the same reference numbers for corresponding parts, the major exception being that the heating of the ribbon 4 is performed by an electromagnetic radiation source 22, e.g. a laser such as a semiconductor diode laser, operable to generate an electromagnetic radiation beam. (In alternative embodiments, source 22 may be a hot air source or an ultrasonic source.) The electromagnetic radiation beam from the radiation source 22 is directed at the ribbon 4 as it passes by an opening in guide 6'. The electromagnetic radiation beam heats and/or induces a reaction in the substance to cause the emission of the trace gas 8. In this configuration, the rollers do not need to be heated, and so they remain relatively cold, obviating the need for heat isolating shaft coupling 18. In a variant, instead of using a radiation source 22, a heated gas stream, such as heated air, may be used; the general objective being to provide some suitable means for heating the ribbon in a stripe across its active width as the ribbon passes by. The mode of heating is of itself not important. An exception to this interchangeability between the use of electromagnetic radiation and heated gas is if a substance requires electromagnetic radiation in a specified wavelength band to undergo a photon-driven chemical reaction such as photo-dissociation.

While the embodiments described in the detailed description envisage the use of either heated gas or electromagnetic radiation as an energy source, other sources of energy may also be used such as intense ultrasound, for example to liberate gas from a medium via intense vibrational energy instead of heat or electromagnetic radiation processes.

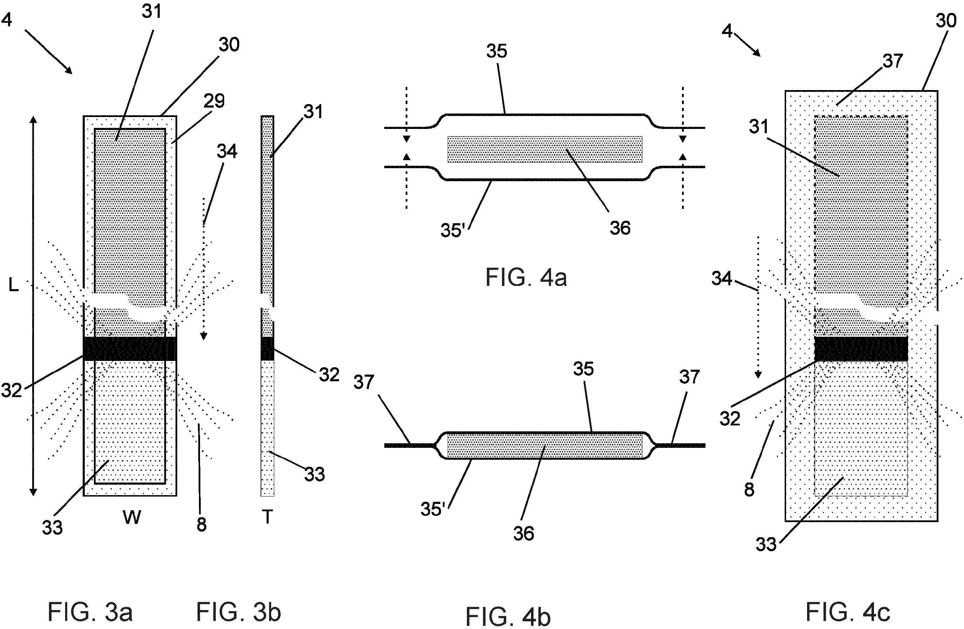

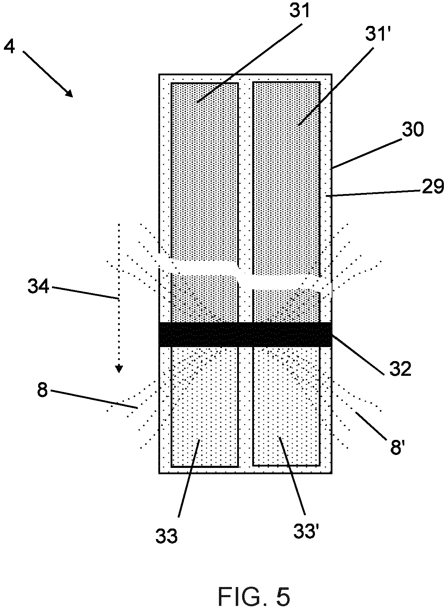

**Figure 3a and 3b** are schematic front and side section views of a first example ribbon medium 4. The ribbon medium 4 comprises porous material infused with an expressible substance or substances. The porous ribbon medium 4 has an extent 30 defined by an indeterminate length 'L', fixed width 'W', and thickness 'T'. The substance is impregnated into the region 31 and bounded by unimpregnated region 29. When rolled up in a tight coil, the region 29 acts as a barrier to unwanted leakage of trace gas which may be slowly but spontaneously emitted at ambient temperature from region 31. When so rolled, the impregnated region 31 only is in contact and exposed to other wraps of region 31, and not exposed to outside air. In one alternative, ribbon medium 4 is of low porosity or non-porous, and is instead coated on at least one of its exterior surfaces. Standard coating techniques may be used employing any of a number of methods including the spraying on and drying of a solvent carrier-based substance, chemical deposition, electroless plating, sputtering, rolling, gas dynamic cold spraying, and many others. These methods and more apply equally to other variations disclosed in association with other figures below. Different substances may be imparted to each side, creating spatially separated gas streams during expression as will be explained in conjunction with Figure 5, below. In another alternative, medium 4 is an impregnated porous material which is also coated with further substance layer(s) on its exterior surface(s), in order to increase areal capacity.

Ribbon medium 4 of Figure 3a, 3b is made to transit an expressor unit 1 in direction 34. As it does so, the substance 31 is heated by rollers 7, 7' or energized by electromagnetic radiation source (laser) 22 (which may be replaced by a heated-gas jet or intense ultrasonic beam in some applications) in an activation zone 32. The ribbon exits zone 32 with depleted substance in exited region 33 after trace gas 8 having been expressed from the substance associated with the medium 4. The lower end of the ribbon shown at 33 is waste and cannot be reused unless optionally recharged.

**Figures 4a, 4b, and 4c** are schematic views of a second example ribbon medium 4 which is devised from an inner cavity layer containing the required expressible substances, with an outer protective envelope. Figures 4b and 4c are schematic front and side section views of the second example ribbon medium, and Figure 4a is a schematic side section view during fabrication of the ribbon medium. A carrier material 36 filled with the substance is laminated between jacketing layers 35, 35'. These outer jacketing layers may conveniently be manufactured from a high temperature melt-resistant (at temperatures of expression) porous thermopolymer film or fabric; during the production of the ribbon medium 4, these layers may be rolled and heat-sealed at appropriately high temperature along their edges 37, or glued or solvent or ultrasonically bonded, thereby laminating porous carrier material 36 within as shown in Figure 4b. Jacketing layers 35, 35' serve to limit spontaneous evaporation or sublimation of substance between the time of manufacture and expression. These layers also act to reduce human contact with the substance during handling, for example during the step of feeding the medium 4 into an expressor apparatus. The layers also impart strength, in particular tensile strength, to the often porous and more fragile carrier material 36, thereby preventing breakage during handling and feeding through an expressor.

During the process of expression, gases 8 are emitted through the porous jacketing 35, 35' to escape as shown in Figure 4c, the jacketing surviving the expression process more or less intact. Examples of high temperature polymers include Kapton^{™} (polyimide) and Polyetherimide (PEI) films, which when perforated up to 10, 20, 40, or 50% by area with micrometer-scale holes provide the porosity needed to allow gas escape. Microporous polypropylene may be used without additional perforations, so long as the temperatures reached during expression do not exceed approximately 150°C. Polypropylene has the additional benefit that the edges 37 may be simply heat-sealed together during production.

In one variation of the design of Figure 4a,b,c, the carrier material 36 comprises a porous material impregnated with substance, such as porous PTFE (polytetrafluoroethylene) tape, which can readily accept a non-polar solvent-carried substance such as an alcohol or paraffin-based substance. The substance is first dissolved in solvent, impregnated as a liquid into the PTFE tape, and then the solvent is evaporated or solidified leaving the substance as a more solid residue inside the pores of the PTFE tape. Other porous materials such as woven fiber and cellulosic materials and so on may also be employed instead of PTFE as less costly alternatives. The solvent may also be aqua or other liquids in some applications. The solvent may be a wax which solidifies at ambient temperature. The fill volume does not necessarily need to be dried prior to the lamination step, indeed the solvent does not necessarily need to be driven off at all, although drying is a very useful step in most cases to prevent leakage during transport, storage and handling, and to prevent solvent from escaping into the transport gas during expression.

In a further variation of the design of Figure 4 a,b,c, the carrier material 36 comprises a low porosity or non-porous tape which is coated with substance on at least one side. The coating methods used may be any one of the types described in association with Figures 3a, 3b, or other methods known in the art of coating. Different substances may be imparted to each side, creating spatially separated gas streams during expression as will be explained in conjunction with Figure 5, below.

In another variation, the carrier material 36 is simply the substance itself, perhaps mixed with a solid inert diluent such as quartz or silica powder to achieve a desired areal density. During manufacture, the outer jacketing layers 35, 35' are sealed together at the edges 37 while substance, diluted or not, is layered within. The substance can also be a liquid which is added to a microporous powder such as silica gel. The resulting mixture can in some formulations resemble a paste.

In the process of manufacturing ribbon medium 4, either of the type shown in Figures 3a, 3b, or 4a, 4b, 4c, the substance can be dispensed onto the medium or carrier material by any usual process such as spraying, liquid dispensing, dipping, powder coating, jet printing (e.g. inkjet nozzle printing), extrusion and the like. These methods also apply to versions where the substance is mixed with a diluent.

**Figure 5** is a schematic front view of a two-stripe ribbon medium 4 containing two different expressible substances in different regions which can be expressed without direct mixing at the time of their simultaneous expression. The two-stripe ribbon medium 4 allows expression of a two-component trace gas, which may be useful, for example, when two gases, or their pre-cursor substances, would chemically react with each other in an unwanted way during a heating or irradiation phase if they were co-located on the medium. For example, if it is desired to express a fragrance and a halogen at the same time, it is generally not desirable to have the two substances in close proximity when stored on the medium or directly on release from the medium, as the two substances will likely cross-react, since halogens are highly reactive species and fragrances are often delicate long-chain organic molecules. The two-stripe ribbon medium 4 as shown comprises a first track 31 and a second track 31', spatially separated, each track containing a different substance.

A single heating or irradiation zone 32 causes respective trace gas species to be expressed from both tracks simultaneously at 8 and 8', but separated by a distance so that at least initially, when the gases are concentrated, dense and hot, they do not mix. This physical separation allows gas cooling and de-concentration (i.e. dilution in the transport gas) to occur before expulsion into an ambient space, thereby limiting the opportunity for chemical cross-reactions between the two trace gases. After expression in zone 32, the tracks exhibit depletion zones 33, 33' as the ribbon moves in direction 34.

The ribbon medium of Figure 5 may be of any of the foregoing constructions described *supra* in conjunction with Figures 3a, 3b, or 4a, 4b, 4c. Furthermore, the design may be augmented with additional tracks if needed for a particular application to provide three or more stripes in a single ribbon medium 4. If the ribbon medium 4 has the substance applied as a surface coating, each of the two sides may contain different substance pairs, allowing two trace gas types per stripe, so potentially 4 trace gas types in a two-stripe ribbon medium 4 as shown.

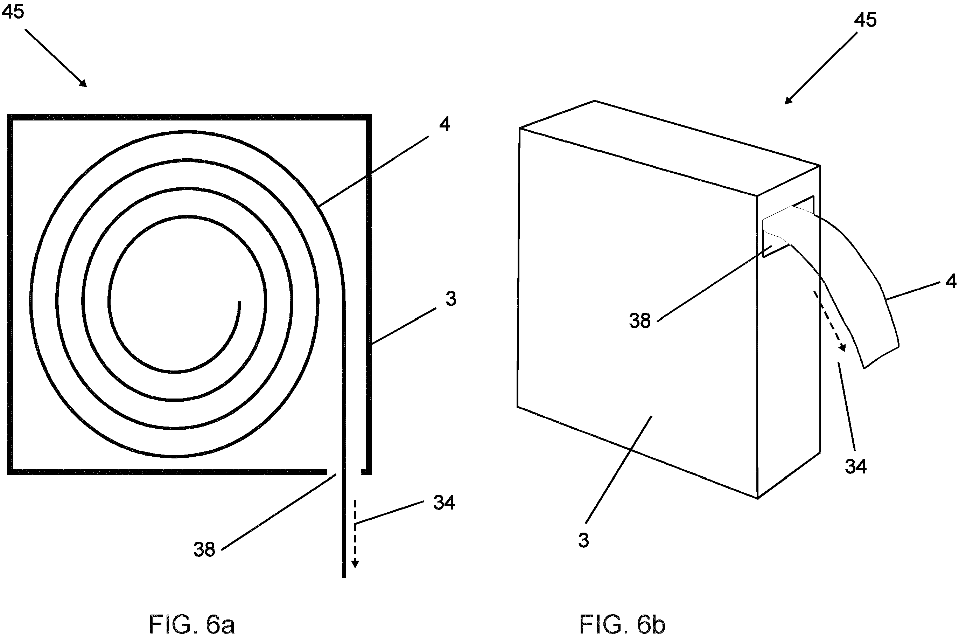

**Figure 6a and 6b** are schematic section and perspective views of a supply-only cartridge 45 according to a first example cartridge, which is suitable for containing ribbon-format media as described above. It is noted that this cartridge type does not take back consumed portions of a ribbon medium. Cartridge 45 holds within it a ribbon medium 4, which may be of either type described above, i.e. comprising single or multiple substance tracks. The ribbon medium 4 is coiled within the cartridge shell 3. An end of the ribbon medium 4 exits a cartridge aperture 38 in direction 34, towards the expressor unit 1. In this design, the shell is 'one way' i.e. it only acts as a source of ribbon to feed an expressor unit 1 and does not serve to accept the return of depleted ribbon medium. The coil may be hub-less as shown, or the coil may be wound on a hub. The use of a hub is not necessary but if included allows the ribbon 4 to be tightly wound so as to reduce spontaneous evaporation or sublimation of substance during shipment and storage or intervals of non-use.

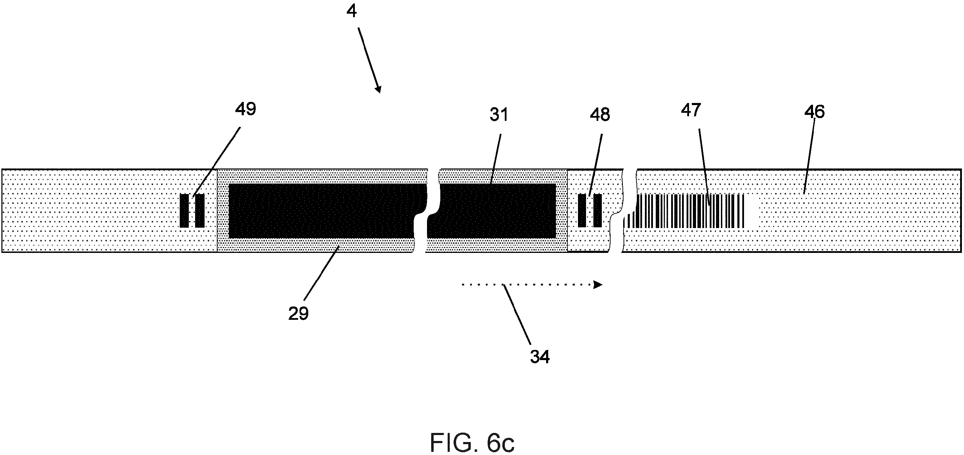

**Figure 6c** is a schematic front view of a ribbon or stick medium 4 showing how additional markings may be provided to encode information that may be used for process control. The ribbon material 46 is factory-impregnated or coated with substance in substance region 31 as previously described. A side margin region 29 either side of the substance region 31 is deliberately left free of substance, i.e. is uncoated or unimpregnated, to form a buffer seal if and when the ribbon is tightly wound on a hub, thereby reducing spontaneous trace gas emission.

One end of the ribbon medium, arranged at a leading end portion of the ribbon medium so as to be the first part of the ribbon medium to be presented to the expressor unit, is provided with a marking 47, e.g. in barcode form, that stores machine-readable information containing relevant general information about the ribbon medium 4. The expressor unit 1 or other part of the air infusion apparatus may include an optical reader 17 for optically reading the barcode (Figure 1a) and transmit the information to a digital controller. Relevant general information may include details of the ribbon, for example, manufacturing date and lot codes, substance type(s) and concentrations, recommended heat or irradiation settings, and so on. As the ribbon medium 4 is fed into the expressor unit 1 in direction 34, the barcode marking 47 may be read by an optical reader 17 (Figure 1a) to inform the digital controller as to the details of the ribbon medium 4. The leading end of the ribbon medium 4 is also provided with a second marking 48, which when scanned by reader 17 signals to the controller that a certain known distance remains to the substance bearing region 31, so that the controller can appropriately activate the heating or irradiation mechanism. In addition, a final marker 49 is provided on the tail end of the ribbon medium to signal to the controller when the ribbon medium is finished and thereby allow the controller to stop the expression process, and feed forward the remaining blank ribbon for disposal.

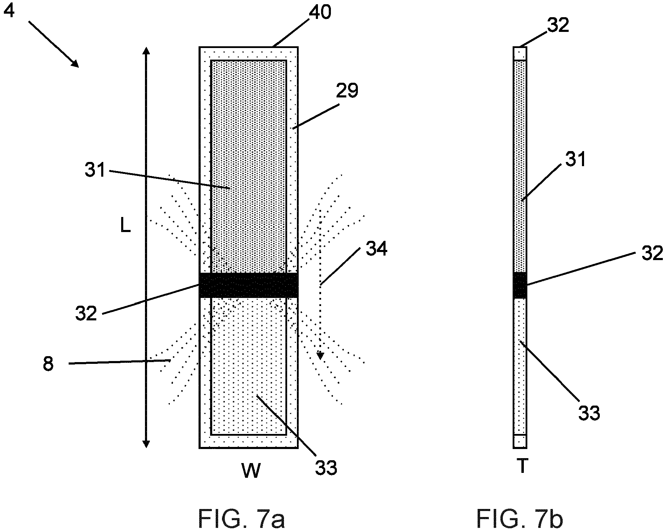

**Figures 7a and 7b** are schematic front and side section views of a stick-format medium 4. The stick-format medium 4 has a more rigid structure than the coilable flexible ribbon-format medium described above. This format of medium operates in a way similar to that described above in conjunction with Figure 3a, i.e., the stick is impregnated with substance, or the stick is coated with substance, or both. The stick medium 4 is fed into the rollers of an expressor in direction 34 during which it emits at least one trace gas 8 from activation zone 32. Unlike the above-described ribbon-format medium, the stick-format medium 4 of Figure 7a, 7b has a limited extent 40 defined by a limited, defined length 'L'. The extent 40 further comprises a defined width 'W' and a thickness 'T'. The thickness 'T' is in most cases thicker than the thickness of the ribbon of Figure 3b in order that the stick-format medium 4 is self-supporting, i.e. rigid or semi-rigid.

While the stick format has an inherently defined and hence limited volumetric or surface area capacity for bearing substance, multiple sticks can be stacked into a multi-stick feeder mechanism (not shown) for feeding sequentially into an expressor unit, leading to much higher capacity. Moreover, if the stick medium 4 is surface coated with substance, the opposing sides of the stick may contain different compounds (not shown) and therefore emit different trace gases as already mentioned above in association with Figure 5. In such a configuration, an expressor unit 1 based on two counter-rotating rollers could be used in which both rollers are heated, but not necessarily equally, so as to cause appropriate, desirable emission of trace gas from each side. Alternatively, the stick medium may be coated or impregnated in dual-track fashion *as per* Figure 5. Stick media 4 may also be impregnated or coated with a substance consisting of a solid wax carrier solvent plus at least one expressible compound. During the step of expression, the wax incrementally transitions to a liquid phase and the trace gas is quickly driven off. As the stick medium emerges from the expressor it cools, the wax then solidifying and trapping within it any residual unexpressed trace gas, thereby preventing an uncontrolled release of trace gas after the expressor is switched off. The stick format of Figures 7a and 7b may also comprise a jacketed carrier material 36 filled with substance in accordance with the description associated with and for the same reasons given in relation to Figures 4a,b,c. Markings may be applied in a similar way to that shown in Figure 6c on one or both sides of the medium, and optically read the same way during the expressor feeding process.

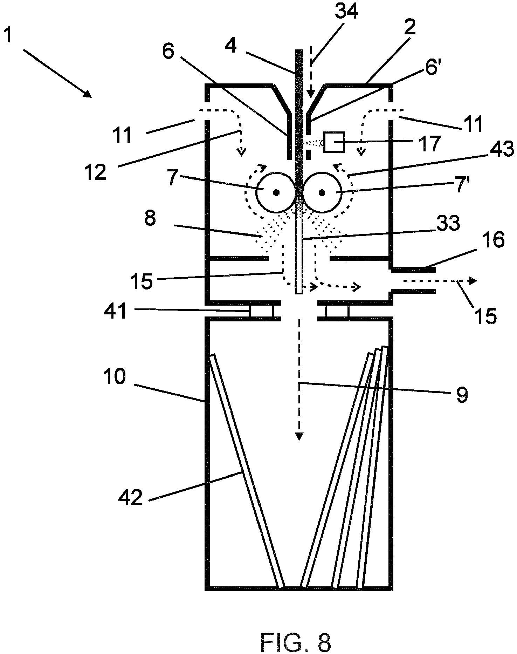

**Figure 8** is a schematic side section view of a third example expressor unit 1 which is designed to accept stick-format media 4 as shown in Figures 7a, 7b. The expressor unit 1 comprises an activator based on a pair of counter-rotating rollers 7, 7'. The expressor unit further includes a waste container 10 arranged to accept spent stick media 4 after they have passed through the activator as schematically shown. The expressor unit 1 may be considered as being composed of two portions. There is an upper portion bounded by housing 2, and a lower portion forming a waste container 10. The upper and lower portions are separated by elastomeric air seal 41. Stick media 4 enter the upper portion in direction 34 through guides 6, 6', which jointly form an input aperture for receiving the stick media 4 by a leading end thereof. A stick medium 4 inserted into the input aperture into contact with the nip region between the rollers 7, 7' to initiate the feed mechanism. In a variant a single roller could be used to nip the stick media 4 against a non-rotating facing surface. The rollers 7, 7' operate similarly to those described above in conjunction with Figures 1a, 1b. An optical sensor 17 may be optionally employed to read markings on the stick media *as per* Figure 6c. In some cases, a second optical sensor may be warranted opposite the sensor 17 to read markings on a second side of stick media.

During the expression process, the rollers rotate in opposing directions as indicated by arrows 43, drawing in the medium; at least one of these rollers is heated. The expressed trace gas 8 emanating from at least one side of the medium enters the surrounding transfer gas in the chamber, the transfer gas being inducted via inlet port 11 and exited via outlet port 15, the motion of the transfer gas being caused by inducing a lower pressure to pertain at the exit of the outlet conduit 16, e.g. by attaching a vacuum pump line to the outlet conduit 16. The transfer gas may also be introduced under elevated pressure at inlet port 11, e.g. using a suitable compressor, although this can be undesirable when dealing with ambient air as the transport gas due to the need to avoid leakage from the chamber when the chamber is under positive pressure. Use of a compressor to generate positive pressure may however be desirable in sealed systems, where the transport gas is furnished from a pressurized bottle and the entire system is sealed within an outer sealed enclosure (not shown). As the stick medium 4 is expressed downwards, it exhibits a substance depletion region 33. This region 33 retains diminishing residual heat causing it to continue to emit trace gases albeit at increasingly lower concentrations the further away it transits from the rollers 7, 7'. The use of a suction pump to transit transfer gas though the chamber removes these residual trace gases through outlet conduit 16. The sum of all gas emissions from the medium 4 is mixed with the transfer gas and exits the outlet conduit 16 as infused gas 15. When the stick medium 4 is fully expressed and has cleared the rollers 7, 7', the stick falls via path 9 by gravity into the waste container 10, where multiple depleted stick media 42 accumulate. Upon a signal from a controller which counts these sticks, or other alert mechanism such as a sight window, the operator may detach waste container 10 and dispose of it in its entirely, thereafter replacing the container with a fresh one. The waste containers may be made of a recyclable material such as pressed, coated cardboard and supplied to the consumer with each package of 'N' stick media, where 'N' may be 10, 20, or 50 sticks or other number of sticks deemed suitable for sale.

The expressor mechanism of Figure 8 may be further augmented with a feeder mechanism (not shown) which resembles the mechanism used by Kodak Carousel^{™} slide projectors, or common card or paper feeders used in modern digital printers. Such a mechanism would sit on top of the housing 2, and automatically inject fresh sticks into the expressor unit when the previous stick was depleted and dropped into waste container 10.

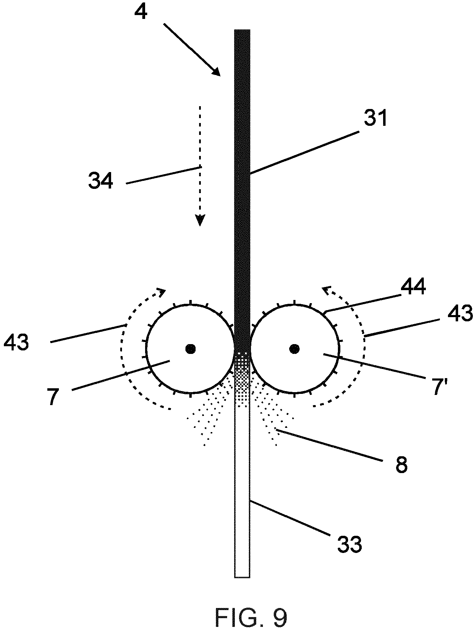

**Figure 9** is a schematic side section view of a pair of counter-rotating rollers 7, 7' as may be used in the expressor of Figure 8, whereby the rollers contain projections for piercing the medium to allow for enhanced expression of the desired trace gas. The rollers 7, 7' of Figure 9 show an adaptation that may be adopted as a variant of what has been described in conjunction with Figures 1a, 1b, 2a, 2b, and in particular Figure 8. In some designs the medium 4 may have a thickness or jacketing which requires or benefits from physical penetration in order to extract the maximum amount of trace gas. Accordingly, spikes 44 in the form of metal or ceramic needles are pressed by the heated rollers into the medium 4 to create openings, e.g. on the micrometer-scale, into the medium to allow gases to escape more efficiently. The spikes may be 10, 20, 40, 60, 80 micrometers in diameter, and 50, 100, 200, 400, 600, 800, 1,000 micrometers in length, or other such length as may be deemed necessary to allow maximal gas expression. In the case of media designs having thin unperforated and non-porous jacketing, the spikes 44 will provide the openings necessary to allow the gases 8 to escape while the stick is heated. The majority of the gas 8 will escape after the spikes are withdrawn from the holes they created, i.e. just below the contact zone 32 between the rollers 7, 7' and the stick 4 as shown. In a variant a single spiked roller could be used to nip the stick media 4 against a facing surface.

In an alternative to spikes, the physical penetration mechanism may be one or more knives, e.g. a series of knives arranged with their blades concentric or parallel to each other, so that the edges of the knife blades partially slot the medium longitudinally as the medium is being fed through, preferably without shredding the medium into multiple strips (not shown). The knife edges may be arranged concentrically and may optionally be heated. Other ways to penetrate the medium and thereby enhance gas escape may be readily envisioned.

**Figures 10a and 10b** are schematic front and side views of a first example air infusion apparatus 50 employing the expressor 1 of Figures 1a and 1b, i.e. for ribbon-format media. Any of the other expressor units for ribbon-format media as described above could be incorporated in the illustrated apparatus. The apparatus 50 is intended for use in distributing trace gas through a human-occupied space such as a room or transportation vehicle. The apparatus is based on a modification of an air cleaner, for example a floor-standing air cleaner having a housing 51 and a fan 53, such as a centrifugal fan, within a cowling 57 and a controller 60 and control panel 61 as is common in the art. Such machines take in air through an inlet grate 55, the air being filtered via filter 56 and expel said air via path 54 through an exhaust port 58, usually located at the top of the machine, creating air output flow 59 into the surrounding space. A great many designs of this general plan are sold on the market today under a variety of brand names.

It is a characteristic of this design that the region between the air filter 56 and the cage fan 53 is under negative pressure, and the region external to the cage fan 53 is under positive pressure. The air infusion apparatus 50 as shown takes advantage of this configuration by using the negative pressure region to suck air downwards from the expressor unit 1 via outlet conduit 16 into the partial pressure region via path 15. As a percentage of the total airflow, this bleed air along path 15 represents a low percentage of the total air expelled through vent 58, but this percentage is more than sufficient for the introduction of trace gases on the order of 0.05, 0.1, 1, 10, 20, or 50ppm concentrations (or other suitable highly dilute concentration) in the expelled air 59. In order to control the concentration of trace gas in the surrounding ambient air, a gas sensor 62 may be optionally installed within the airflow path to monitor said concentration and allow the controller 60 to regulate the introduction of additional trace gas by controlling the consumption rate of medium within expressor 1.

In some cases where the trace gas is filtered by the filter 56, the gas sensor 62 requires direct access to ambient air via a separate pathway around the filter (not shown). Since the amount of air required by such a gas sensor is tiny (picoliters per second), lack of filtration of this air is not consequential to the overall performance of the air cleaning function. In addition to the features noted above, the apparatus 50 also has a waste container access door 52 for the purpose of allowing the user to install and remove the waste container 10. A cover lid 21 conceals and protects the input to the expressor 1, the cover containing perforations 11' which allow air intake into the expressor when the lid 21 is closed.

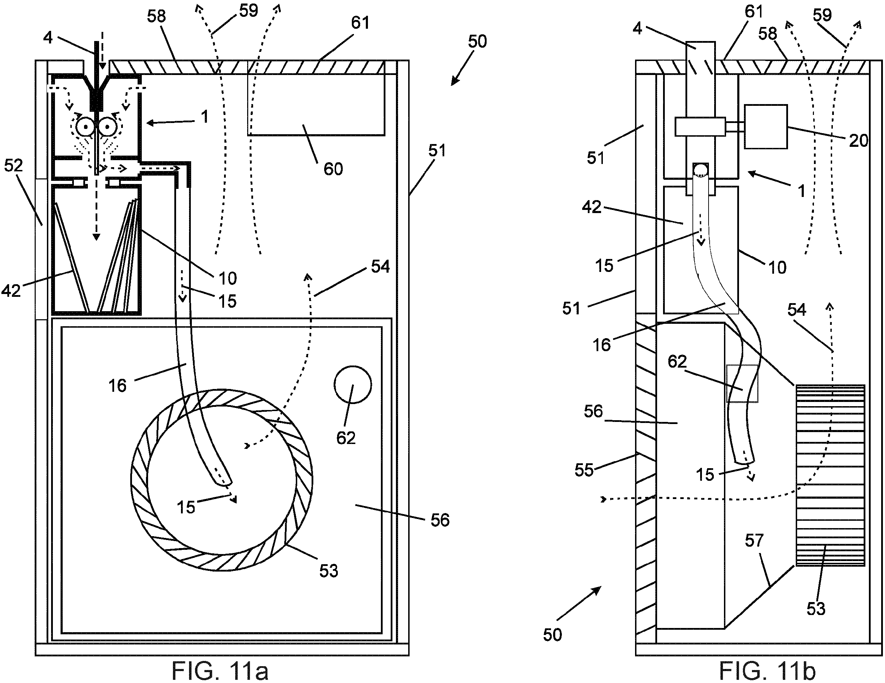

**Figures 11a and 11b** are schematic front and side views of a second example air infusion apparatus 50 employing the expressor 1 of Figure 8, i.e. for stick-format media. Compared with Figures 10a and 10b, this configuration is nominally simpler in design, especially as regards the need for an enlarged entry to the expressor and a lid in conjunction with Figures 10a and 10b.

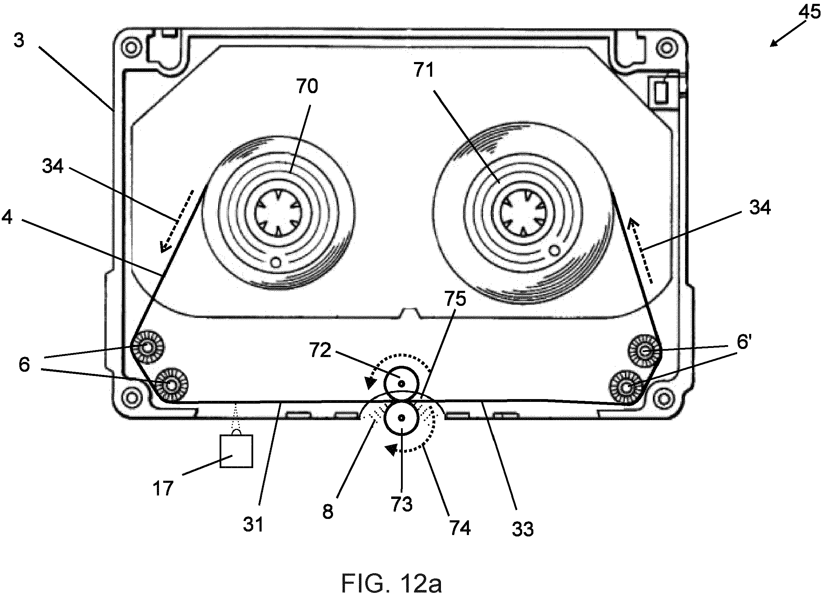

**Figure 12a** is a schematic front section view of a second example cartridge 45 which has a design based on a modification to a conventional audio cassette introduced in 1963. Other designs having the same basic features are of course possible. One can think of other tape cartridge or cassette designs implemented over the past decades for audio and digital tape storage, from 8-track audio cartridges (recirculating tape) to wide-format digital media, and modifications thereof. As is notoriously well known, a cassette of this general kind is characterized by first and second reels between which a tape is fed via counter-rotating rollers past a magnetic read/write head. A general characteristic of these designs which can be put to good use is that a tape of undefined length (subject to a maximum) is provided on one hub, and as the tape is wound off that hub, it is wound onto the other hub, both spools being contained within a common housing. In the cassette-format cartridge 45 according to this example, a ribbon-format medium is contained in shell 3 and is initially wound on a first reel. In use, the medium is fed through an expressor (at the position of the read/write head of a conventional cassette) and then the expressed medium is wound onto a second reel. In Figure 12a, the expressor is based on a counter-rotating pair of rollers, at least one of which is heated.

This design allows the construction of media housings which obviate the need for the user to handle the ribbon-format medium in any way, thereby allowing the medium potentially to contain compounds that, if the medium were touched, may be an irritant to humans in the concentrations necessary for efficacy.

Figure 12a shows a tape supply hub 70 around which is spooled impregnated or coated media 4 which is pulled along path 34 via guide rollers 6, 6', to take-up hub 71. At the location where normally a magnetic read/write head would be located, a heated roller 73 (not part of the cassette) is introduced by the drive mechanism (not shown) into contact with the tape 4, pressing against a passive roller 72 in rotational direction 74, the roller 72 being built into the cassette housing itself. Optical reader 17, optionally may be employed to read tape marking information as described in conjunction with Figure 6c above. The cassette housing incorporates a cut-out region 75 in the area where the tape is nipped between the two rollers, the cut-out serving to allow the trace gases 8 to be evacuated by the drive mechanism, described below.

As the impregnated or coated tape medium 4, the portion 31 containing substance, passes between the two pressure rollers 72, 73, trace gas 8 is expressed into the surrounding space in reaction to the heat supplied by roller 73. Depleted tape section 33 is thereafter rolled up on to take-up hub 71 in due course. At the end of the supply of usable un-depleted tape 31, the entire cassette can be disposed of preferably by recycling.

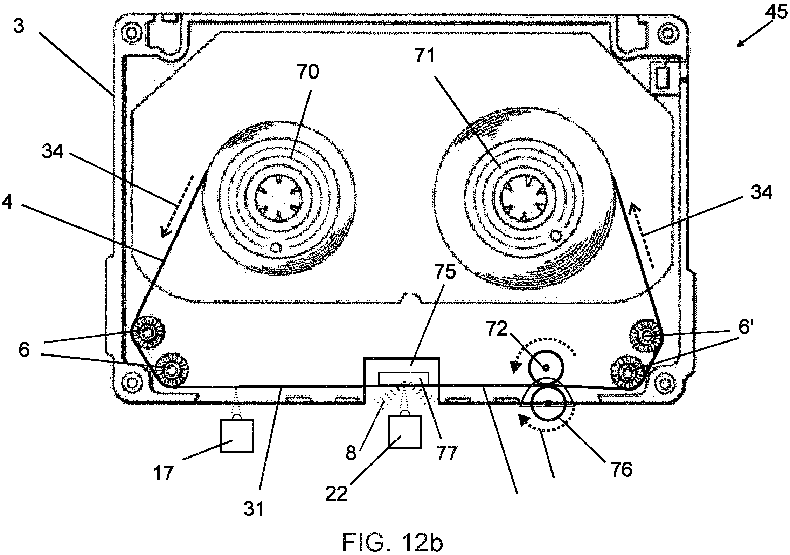

**Figure 12b** is a schematic front section view of a third example cartridge, which may be considered to be a modification of the design of Figure 12a, in which the expressor is based on an electromagnetic radiation source instead of heated rollers. Compared with Figure 12a, the roller 72 is moved to one side and an unheated pressure drive roller 76 is arranged to engage the tape and press into roller 72, so that a counter-rotating roller pair 72, 74 is formed that controllably feeds a ribbon-medium tape on to a take-up hub 71. The expression of gas 8 occurs under the influence of an electromagnetic radiation source, such as a laser 22, directed at tape medium 4. A backing pad 77 stabilizes the location of the tape with respect to the radiation source 22, the pad being porous or open enough to allow inward-escaping gases to circulate in the region of cut-out 75, from whence the gas may be further extracted as will be described below.

Figures 12a and 12b represent single-use cassettes, the medium is not intended to be rechargeable. The roller 73 of Figure 12a may be of the type described in conjunction with Figure 9 to enhance gas expression in the case of insufficiently porous tapes, for example if the tapes are impregnated.

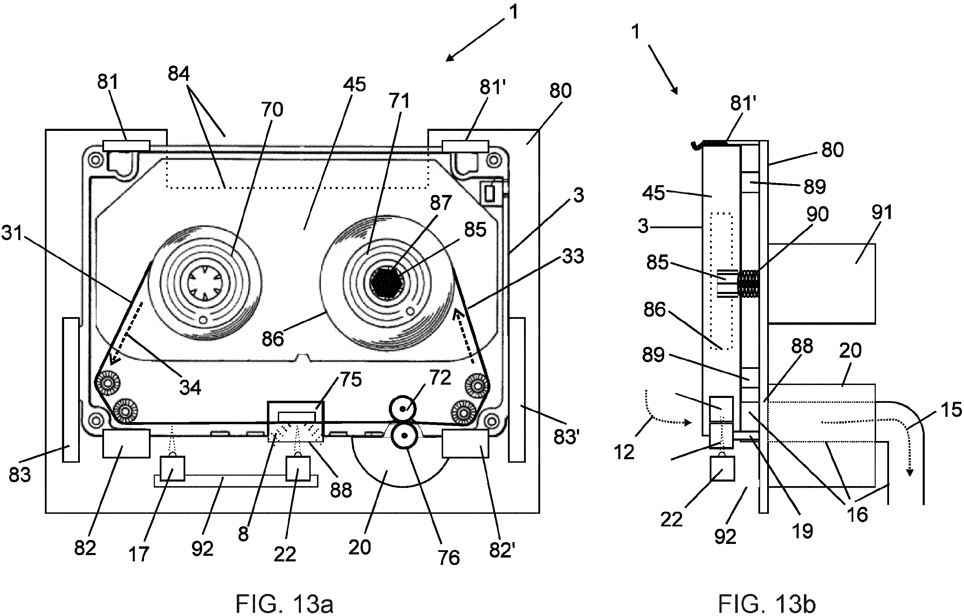

**Figures 13a and 13b** are schematic front section and side section views of an example expressor 1 for the cartridge 45 of Figure 12b but may be adapted readily for the cartridge of Figure 12a. The expressor drive mechanisms for media cassettes 45 strongly resemble the designs used for audio cassette tapes, with key modifications as shown here. As with audio cassette drive mechanisms, a tape regulating drive motor 20 drives roller 76 via shaft 19 to control the feed rate of the tape medium 4. In the case of the expressor 1, this motor is conveniently and preferably a speed controllable type such as a phase-controlled stepper motor. A take-up motor 91 controls the spooling uptake of depleted media 33 onto hub 71 via drive pinion 85, which engages reel hub 87 under pressure supplied by pinion extension spring 90. The extent of the tape take-up is shown at 86, this of course being variable. Motor 91 may be an ordinary DC motor as the role of this motor is only to provide a variable torque to the hub 71, and does not need to be precisely controlled, but the torque requirement depends on the diameter of extent 86; the larger this diameter, the more torque the motor 91 needs to supply in order to provide for relatively constant tension on the tape past the rollers 72, 76. The controller of this expressor drive (not shown) by design calculates the amount of motor current necessary for this torque based on the consumption state of the media.

Radiation source 22, optical reader 17, and other features are as described *as per* Figure 12b. Additional mechanical features shown include mounting and backing plate 80, support 92 for the optical and radiation devices, mounting and positioning blocks 82, 83 and 89, retaining clips 81, 81' which are lateral to opening 84. Gas outlet conduit 16 heads into mounting plate 80 at plate cut-out 88 proximal where the trace gases 8 are expressed, cut-out 88 being generally co-terminus with cassette cut-out 75. The suction supplied by the mouth of gas outlet conduit 16 at cut-out 88 removes the trace gases produced in this region and transports them, along with the transport gas (air), along path 15. The cut-outs 75 and 88 are large enough to encompass both the front and rear regions of the tape where the gas is being expressed. Loading the cassette into the aforementioned expressor drive mechanism involves sliding the cassette downwards at a slight angle into the two positioning blocks 82, 82' then snapping the top of the cassette into retaining clips 81, 81'. The cut-out in the mounting plate 80 at 84 facilitates this process. Unloading is accomplished by merely reversing the action of loading.

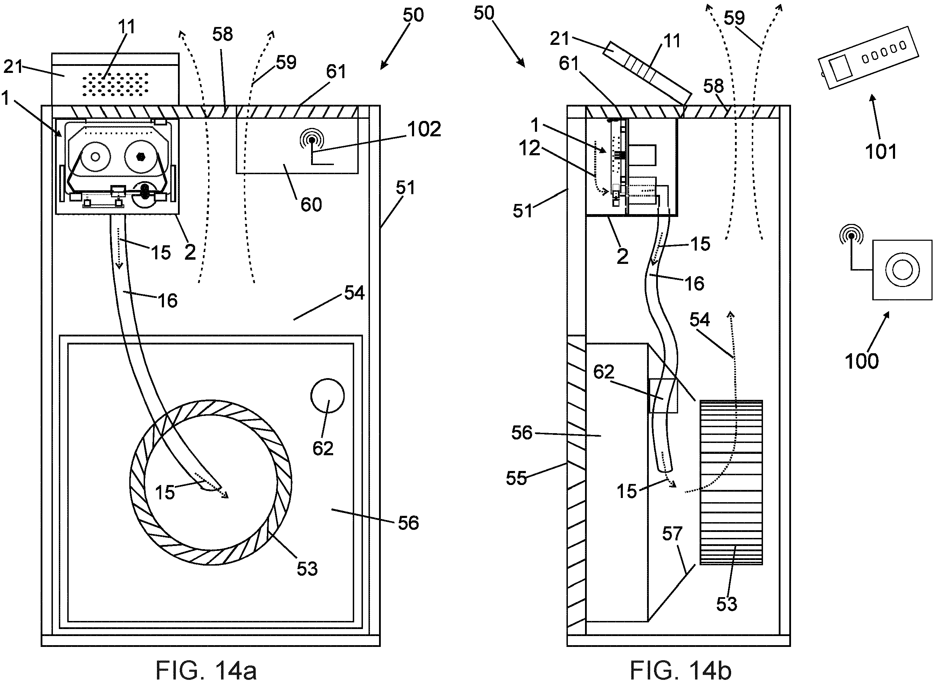

**Figures 14a and 14b** show a variation of the apparatus as discussed in conjunction with Figures 11a, 11b, adapted for use with the expressor design of Figures 13a, 13b. In addition, and unrelated to the expressor itself, is a sensing feature for the remote detection of trace gas via remote radio-linked sensor 100 and internal receiver with antenna 102. One or more remote sensors 100 facilitates more accurate control of the trace gas in a volume of space distant from the apparatus 50, allowing the controller 60 to adapt more accurately the expression rate of trace gas from expressor 1. Such a sensor or sensors can be used in place of, or in addition to, built-in gas sensor 62. Figure 14b further illustrates a remote control 101 which can be conveniently used by an operator to input commands to controller 60, as is customary in the art of air handling devices. Furthermore, the controller 60 may also employ radio control linking the apparatus to a portable control device such as a smart phone, tablet, notebook, or other personal computing device as is well known among innumerable modern consumer applications. Such control can be employed to provide more sophisticated timing and control operations such as delayed start, scheduled start/stop, gas concentration profiles with respect to time, media consumption control, end-of-media warnings and the like. In some situations, it is desirable to provide for user restrictions to prevent control or supervision by unauthorized personnel; this too can be accomplished via a personal computing device such as a smart phone.

### REFERENCE NUMERALS

- 1: Expressor unit / trace gas emission apparatus
- 2: Expressor unit enclosure
- 3: Media cartridge shell
- 4: Substance-bearing strip-format carrier
- 5: Media path input to rollers
- 6: Media guides
- 6': Media guides
- 7: Pressure roller
- 7': 2nd Pressure roller
- 8: Gas emissions
- 8': Gas emissions - second
- 9: Media path, expended media

- 10: Waste container for media
- 11: Air intake openings
- 11': Air intake openings, additional
- 12: Airflow, inducted into 1
- 13: Perforated separator upper
- 14: Perforated separator lower
- 15: Gas-infused air path
- 16: Infused air outlet conduit
- 17: Optical code/mark sensor
- 18: Heat isolator shaft coupling
- 19: Motor drive shaft

- 20: Rotary drive (stepper motor)
- 21: User access cover
- 22: Electromagnetic radiation source / hot air source / ultrasonic source
- 23: Nip region between rollers
- 29: Barrier zone

- 30: Media extent (L x W) arbitrary
- 31: Un-depleted media region of track 1
- 31': Un-depleted media region of track 2
- 32: Activation zone of carrier
- 33: Depleted media region
- 33': Depleted media region, right side
- 34: Direction of media travel
- 35: Media envelope, upper
- 35': Media envelope, lower
- 36: Media substance fill volume / carrier material
- 37: Envelope bonding zone
- 38: Exit aperture of cartridge shell

- 40: Media extent (L x W) fixed
- 41: Air Seal between 2 and 10
- 42: Spent media
- 43: Rotation direction of rollers
- 44: Roller spikes
- 45: Tape-dispensing cartridge
- 46: Media leader section
- 47: Bar code
- 48: Start mark
- 49: End mark

- 50: Air infusion apparatus / gas dispenser unit
- 51: Apparatus housing
- 52: Waste container access door
- 53: Centrifugal (cage) fan
- 54: Airflow through fan
- 55: Air inlet/intake grille
- 56: Air inlet/intake filter
- 57: Air inlet/intake cowling
- 58: Air outlet/exhaust
- 59: Outlet/exhaust air through exit vent

- 60: Electronics
- 61: User interface panel
- 62: Gas sensor

- 70: Dispensing reel
- 71: Return reel
- 72: Roller passive built into cassette
- 73: Roller driven, heated
- 74: Roller rotation direction
- 75: Aperture for airflow
- 76: Drive roller, unheated
- 77: Backing pad, porous

- 80: Mounting plate
- 81: Spring clip
- 81': Spring clip
- 82: Cartridge slotting brackets
- 82': Cartridge slotting brackets
- 83: Side brackets
- 83': Side brackets
- 84: Mounting plate cut-out
- 85: Take-up reel drive pinion
- 86: Take-up media extent
- 87: Take-up reel hub
- 88: Vent cut-out in mounting plate
- 89: Media case standoff spacers

- 90: Pinion extension spring
- 91: Take-up hub motor
- 92: Laser & optics support

- 100: Trace gas sensor
- 101: Controller
- 102: Internal antenna & receiver

## Claims

1. A trace gas emission apparatus for processing a substance-bearing, strip-format carrier to activate the substance and thereby release a trace gas, the apparatus comprising:
a substance activator defining an activation zone dimensioned to accommodate a length portion of a strip-format carrier, the substance activator being configured to energize a length portion of a carrier situated in the activation zone and thereby activate substance in that length portion; and
a feed mechanism configured to feed a strip-format carrier through the activation zone from one end of the strip-form carrier to another, thereby to enable the substance activator to effect incremental release of trace gas from respective length portions of the strip-format carrier.

2. The apparatus of claim 1, wherein the substance activator comprises an electromagnetic radiation source operable to direct an electromagnetic radiation beam to the activation zone.

3. The apparatus of claim 1, wherein the substance activator comprises a heater arranged to apply heat in the activation zone.

4. The apparatus of claim 3, wherein the heater comprises a hot gas source operable to direct a hot carrier gas stream to the activation zone.

5. The apparatus of claim 3, wherein the substance activator comprises a roller arranged in the activation zone to come into contact with a length portion of the strip-format carrier, wherein the heater is operable to heat the roller so that substance activation takes place by heat transfer from the roller to a contacted length portion of the strip-format carrier.

6. The apparatus of claim 5, comprising a further roller that is arranged with said roller to form a counter-rotating roller pair defining a nip region, wherein the feed mechanism is configured to convey the strip-format carrier through the nip region.

7. The apparatus of claim 6, wherein the heater is operable to heat the further roller.

8. The apparatus of claim 5, 6 or 7, wherein the feed mechanism comprises a rotary drive connected to drive at least one of the roller and the further roller, so that the counter-rotating roller pair fulfills a dual function for both the feed mechanism and the substance activator.

9. The apparatus of claim 1, 2, 3 or 4, wherein the feed mechanism comprises a roller and a further roller arranged to form a counter-rotating roller pair defining a nip region and a rotary drive connected to drive at least one of the roller and the further roller so as to convey the strip-format carrier through the nip region.

10. The apparatus of any preceding claim, wherein the feed mechanism is configured to feed:
a flexible tape as the strip-format carrier, and the substance activator is configured to activate the substance from a length portion of the flexible tape; or
a self-supporting stick as the strip-format carrier, and the substance activator is configured to activate the substance from a length portion of the self-supporting stick.

11. The apparatus of any preceding claim, further comprising a controller configured to control at least one of the substance activator and the feed mechanism so that the trace gas is emitted at a controlled rate, the apparatus optionally further comprising a sensor configured and arranged to measure concentration of the trace gas and to supply a sensor signal indicative thereof to the controller, the controller being configured to control at least one of the substance activator and the feed mechanism responsive to the sensor signal so as to maintain the concentration of the trace gas at a desired level.

12. A tape dispensing cartridge comprising:
a tape wound in a coil, the tape storing at least one substance capable of producing a trace gas, wherein the at least one substance is retained bound to the tape in ambient conditions and in response to being locally activated emits the trace gas; and
a shell in which the coil of tape is rotatably arranged, the shell having an aperture from, or past, which the tape is conveyable under rotation of the coil.

13. The cartridge of claim 12, further comprising a dispensing reel that is rotatably mounted in the shell and fixed to one end of the tape and optionally further comprising a return reel that is rotatably mounted in the shell and fixed to another end of the tape, such that the tape can be wound from the dispensing reel onto the return reel, the tape having a length portion situated between the dispensing reel and the return reel that lies at the aperture.

14. A gas dispenser unit for dispensing a trace gas into an environment, the apparatus comprising:
an air inlet providing gas communicative access to the apparatus from its surroundings;
an air outlet providing gas communicative access from the apparatus to its surroundings;
a fan which generates an air flow through the apparatus along a flow path from the air inlet to the air outlet; and
a trace gas emission apparatus according to any one of claims 1 to 11 arranged to emit trace gas into the flow path generated by the fan so that air emitted from the air outlet is infused with the trace gas.

15. The unit of claim 14, further comprising a trace gas dispensing conduit having an inlet aperture arranged to receive trace gas emitted from the trace gas emission apparatus and an outlet aperture arranged to emit trace gas into the air flow ahead of the fan.
